# EUROPÄISCHE PATENTSCHRIFT

(11) **EP 2 511 259 B1**
(45) Veröffentlichungstag und Bekanntmachung des Hinweises auf die Patenterteilung: **21.10.2015**
(21) Anmeldenummer: 12172693.9
(22) Anmeldetag: 12.12.2007
(51) Int. Cl.: C07C 263/10, C07C 265/14

(54) **Verfahren zur Herstellung von Isocyanaten**
Method for making isocyanates
Procédé destiné à la fabrication d'isocyanates

(30) Priorität: 17.01.2007 EP 07100644
(43) Veröffentlichungstag der Anmeldung: 17.10.2012
(62) Teilanmeldung aus: 07857437.3
(73) Patentinhaber: BASF SE, 67056 Ludwigshafen (DE)
(72) Erfinder: Wölfert, Andreas, 74906 Bad Rappenau (DE); Knösche, Carsten, 67150 Niederkirchen (DE); Mattke, Torsten, 67251 Freinsheim (DE); Stroefer, Eckhard, 68163 Mannheim (DE)

(56) Entgegenhaltungen:
- EP-A- 0 570 799
- EP-A- 0 593 334
- WO-A-01/00569
- WO-A-2004/056758
- WO-A-2005/123665
- US-A- 3 331 873
- "Römpps Chemie Lexikon", Römpp Online, Version 3.12 * Brom *

## Beschreibung

Die vorliegenden Erfindung betrifft ein Verfahren zur Herstellung von Diisocyanaten aus Diaminen und Phosgen in der Gasphase.

EP 570799, Beispiel 1 beschreibt die Aufarbeitung eines durch Gasphasenphosgenierung erhaltenen Reaktionsgemisches mittels eines mit Wasser berieselten Waschturms zur Abtrennung von Phosgen und Chlorwasserstoff.

Durch eine derartige Aufarbeitung werden überschüssiges Phosgen sowie Chlorwasserstoffgas vernichtet und können nicht mehr nutzbringend in die Reaktion eingesetzt werden.

EP 593334 B1 und EP 699657 B1 offenbaren die Möglichkeit Phosgen oder Chlorwasserstoffgas zu verwerten oder zu zerstören, ohne jedoch auf die spezielle Problematik von rückgeführtem Phosgen einzugehen.

EP 749 958 B1 Absatz [0018] und EP 1078918 B1 Absatz [0018] erwähnen die Möglichkeit, nach Beendigung einer Gasphasenphosgenierung von Triaminen überschüssiges Phosgen rückzugewinnen und dabei wiedergewonnenes Chlorwasserstoffgas wieder in die Phosgensynthese einzusetzen.

Auch hier werden keine Angaben gemacht, die das rückgeführte Phosgen näher beschreiben.

US 4,581,174 beschreibt die kontinuierliche Herstellung von organischen Mono- und/oder Polyisocyanaten durch Phosgenierung des primären Amines in einem Mischkreis unter teilweiser Rückführung der isocyanathaltigen Reaktionsmixtur, wobei der HCl-Anteil in der rückgeführten Mischung kleiner als 0,5 % ist. Auch hier gilt, dass die kontinuierliche Rückführung des Isocyanates in die Reaktionszone mit freiem Amin die Harnstoffbildung fördert. Der ausfallende Harnstoff gefährdet den stabilen Betrieb des Verfahrens.

GB 737 442 beschreibt die Rückgewinnung von Phosgen aus der Isocyanatsynthese. Das rückgewonnene Phosgen hat einen HCI-Gehalt von 0,5 bis 0,7 %.

WO 2005/123665 beschreibt eine Gasphasenphosgenierung mit einem reduzierten Strömungsquerschnitt.

DE 10261191 A1 und WO 2004/58689 beschreiben Phosgenierungen, bei denen der HCl-Gehalt im phosgenhaltigen Eduktstrom weniger als 0,4 bzw. mehr als 0,8 Gew% enthält.

Diese Schriften differenzieren in der Problematik nicht zwischen Gas- und Flüssigphasenphosgenierung und beziehen sich bevorzugt nur auf Flüssigphasenphosgenierung.

Nachteilig an all diesen Verfahren ist, daß der Chlorgehalt im Phosgen bzw. im Verlauf der Phosgenierung unberücksichtigt bleibt.

Die internationale Anmeldung mit dem Aktenzeichen WO 2007/014936 (PCT/EP2006/064850) und dem Anmeldedatum 31.07.2006 beschreibt ein Gasphasenphosgenierungsverfahren, in dem der Gehalt an Chlorwasserstoff unterhalb einer bestimmten Schwelle bleiben sollte.

WO 04/56758 erwähnt Chlor als Bestandteil von Phosgen in einer breiten Liste von Nebenkomponenten, offenbart jedoch keine Lehre über die spezielle Problematik des Chlorgehalts in einer Gasphasenphosgenierung.

US 3331873 offenbart ein allgemeines Verfahren zur Abtrennung von Chlor aus Phosgen mit Hilfe von Aktivkohle auf einen Gehalt von weniger als 25 ppm.

In dem offenbarten Verfahren wird kein Hinweis auf die spezielle Problematik in Phosgenierungen und insbesondere in Gasphasenphosgenierungen gegeben.

WO 01/00569 beschreibt die Wirkung des Gehalts an Brom und bromhaltigen Verbindungen auf die Farbzahl bei Flüssigphasenphosgenierungen unter einem Druck bis 100 bar und einer Temperatur von 0 - 130 °C.

In dem offenbarten Verfahren wird kein Hinweis auf die spezielle Problematik in Gasphasenphosgenierungen gegeben.

Die Gasphasenphosgenierung wird üblicherweise bei Temperaturen von 200 bis 600 °C durchgeführt. Aufgrund dieser hohen Temperaturen ergeben sich besondere Anforderungen an die Ausführung des Verfahrens, um einen dauerhaften Betrieb des Verfahrens ohne Leckagen durch erhöhte Material- und besonders Reaktorwandbeanspruchung im Hochtemperaturbereich zu erreichen.

Durch die hohen Temperaturen in Kombination mit den korrosiven Reaktionsmedien ergeben sich spezielle Anforderungen an das Verfahren und die eingesetzten Werkstoffe. So ist beispielsweise bekannt, dass bei hohen Temperaturen (ab ca. 400 °C) Phosgen autokatalytisch in molekulares Chlor (Cl₂) und Kohlenstoffmonoxid (CO) dissoziiert. Chlor führt bei hohen Temperaturen zu Werkstoffversprödungen, vermutlich durch Einlagerung in den Werkstoff. Derartig versprödete Materialien und insbesondere Reaktorwandungen können aber z.B. bei unvermeidlichen Vibrationen in der Pro-duktionsanlage belastet werden und reissen bzw. brechen, so dass die Wahrscheinlicheit von Leckagen erhöht wird. Zudem kann Chlor mit unlegierten Stählen oberhalb von 170 °C exotherm in einem sogenannten Chlor-Eisen-Brand reagieren. Dies ist insbesondere bei der Handhabung des sehr giftigen Phosgens technisch problematisch.

Aufgabe der Erfindung war es daher, ein Verfahren bereit zu stellen, das in der Umsetzung von Diaminen mit Phosgen in der Gasphase zu den korrespondierenden Diisocyanaten und Chlorwasserstoff (HCI) eine Reaktionsführung zuläßt, in der Werkstoffversprödungen verringert werden können.

Die Aufgabe wurde gelöst durch ein Verfahren zur Herstellung von Diisocyanaten durch Umsetzung der korrespondierenden Diamine mit Phosgen im stöchiometrischen Überschuß von Phosgen in mindestens einer Reaktionszone,
wobei man die Reaktionsbedingungen so wählt, daß zumindest die Reaktionskomponenten Diamin, Diisocyanat und Phosgen bei diesen Bedingungen gasförmig sind, und wobei man der Reaktionszone mindestens einen diaminhaltigen und mindestens einen phosgenhaltigen Gasstrom zuführt,
wobei der Massenbruch von Brom im phosgenhaltigen Strom weniger als 50 Gew.ppm beträgt.

Bei der Gasphasenphosgenierung ist es erfindungsgemäß anzustreben, daß die im Reaktionsverlauf auftretenden Verbindungen, also Edukte (Diamin und Phosgen), Zwischenprodukte (insbesondere die intermediär entstehenden Mono- und Dicarbamoylchloride), Endprodukte (Diisocyanat), sowie gegebenenfalls zudosierte inerte Verbindungen, unter den Reaktionsbedingungen in der Gasphase verbleiben. Sollten diese oder andere Komponenten sich aus der Gasphase z.B. an der Reaktorwand oder anderen Apparatebauteilen abscheiden, so kann durch diese Abscheidungen der Wärmeübergang oder die Durchströmung der betroffenen Bauteile unerwünscht verändert werden. Dies gilt insbesondere für auftretende Aminhydrochloride, die sich aus freien Aminogruppen und Chlorwasserstoff (HCI) bilden, da die resultierenden Aminhydrochloride leicht ausfallen und nur schwer wieder verdampfbar sind.

In einer bevorzugten Ausführungsform kann zusätzlich zu einem verringerten Chlorgehalt der Massenbruch von Chlorwasserstoff im phosgenhaltigen Strom nach einer gegebenenfalls durchgeführter Vermischung mit Frisch-Phosgen vor der Vermischung mit dem aminhaltigen Strom weniger als 15 Gew.-%, bevorzugt weniger als 10, besonders bevorzugt weniger als 5 Gew% betragen.

Dadurch wird erreicht, daß die Bildung von Aminhydrochloriden entscheidend verringert werden kann, so daß die Gefahr von Belagsbildungen im Reaktor reduziert wird.

Durch den geringen Gehalt an Chlor, d.h. in dieser Schrift molekularem Chlor (Cl₂), im in die Reaktion eingespeisten Phosgen ist es möglich, den gesamten Chlorgehalt im Verlauf der Umsetzung des Diamins zum korrespondierenden Diisocyanat trotz des im Verlauf der Reaktion durch Spaltung von Phosgen entstehenden Chlors so gering wie möglich zu halten, so daß die Gefahr der Werkstoffversprödung und/oder Chlor-Eisen-Bränden verringert oder sogar ausgeschlossen werden kann.

Der Chlorgehalt im phosgenhaltigen Strom kann nach einer gegebenenfalls durchgeführter Vermischung mit Frisch-Phosgen vor der Vermischung mit dem aminhaltigen Strom weniger als 1000 Gew.ppm, bevorzugt weniger als 500, besonders bevorzugt weniger als 250, ganz besonders bevorzugt weniger als 100, insbesondere weniger als 50 und speziell weniger als 25 Gew.ppm betragen

Zusätzlich oder alternativ dazu beträgt der Gehalt an Brom oder Iod oder deren Gemische in molekularer oder gebundener Form im phosgenhaltigen Strom nach einer gegebenenfalls durchgeführten Vermischung mit Frisch-Phosgen vor der Vermischung mit dem aminhaltigen Strom weniger als 50 Gew.ppm, bevorzugt weniger als 40 Gew.ppm, 35 Gew.ppm, 30 Gew.ppm oder 25 Gew.ppm oder weniger, insbesondere von 10 Gew.ppm oder weniger und speziell von 5 Gew.ppm, 3 Gew.ppm, 2 Gew.ppm oder 1 Gew.ppm oder weniger.

Unter Brom oder Iod in molekularer Form werden im Rahmen des vorliegenden Textes solche Moleküle verstanden, die lediglich aus Brom- oder Iodatomen bestehen. Unter Brom oder Iod in gebundener Form werden Moleküle verstanden, die neben Brom oder Iod noch weitere, von den genannten Atomen jeweils verschiedene Atome enthalten. Wenn nicht anders bezeichnet, wird in dem vorliegenden Text unter dem Begriff "Brom" molekulares Brom (Br₂) verstanden.

Gemäß dem erfindungsgemäßen Verfahren erfolgt die Umsetzung von Phosgen mit Diamin in der Gasphase.

Diisocyanate, die mit dem erfindungsgemäßen Verfahren hergestellt werden können, können aromatische, cycloaliphatische oder aliphatische Isocyanate sein.

Cycloaliphatische Isocyanate sind solche, die mindestens ein cycloaliphatisches Ringsystem enthalten.

Aliphatische Isocyanate sind solche, die ausschließlich Isocyanatgruppen aufweisen, die an gerade oder verzweigte Ketten gebunden sind.

Aromatische Isocyanate sind solche, die mindestens eine an mindestens ein aromatisches Ringsystem gebundene Isocyanatgruppe aufweisen.

(Cyclo)aliphatische Isocyanate steht im Rahmen dieser Anmeldung kurz für cycloaliphatische und/oder aliphatische Isocyanate.

Beispiele für aromatische Diisocyanate sind sind bevorzugt solche mit 6 - 20 C-Atomen, beispielsweise monomeres Methylen-di(phenylisocyanat (MDI), 2,4- und/oder 2,6-Toluylendiisocyanat (TDI) und Naphtyldüsocyanat (NDI).

Diisocyanate sind bevorzugt (cyclo)aliphatische Diisocyanate besonders bevorzugt (cyclo)aliphatische Diisocyanate mit 4 bis 20 C-Atomen.

Beispiele für übliche Diisocyanate sind aliphatische Diisocyanate wie Tetramethylendiisocyanat, Pentamethylendiisocyanat (1,5-Diisocyanatopentan), 2-Methyl-1,5-diisocyanatopentan, Hexamethylendiisocyanat (1,6-Diisocyanatohexan), 1,8-Octamethylendiisocyanat, 1,10-Decamethylendiisocyanat, 1,12-Dodecamethylendiisocyanat, 1,14-Tetradecamethylendiisocyanat, Derivate des Lysindiisocyanates, Tetramethylxylylendiisocyanat (TMXDI), Trimethylhexandiisocyanat oder Tetramethylhexandiisocyanat, sowie 3 (bzw. 4), 8 (bzw. 9)-Bis(isocyanatomethyl)-tricyclo[5.2.1.0^{2.6}]decan-lsomerengemische, sowie cycloaliphatische Diisocyanate wie 1,4-, 1,3- oder 1,2-Diisocyanatocyclohexan, 4,4'- oder 2,4'-Di(isocyanatocyclohexyl)methan, 1-Isocyanato-3,3,5- trimethyl-5-(isocyanatomethyl)cyclohexan (Isophorondiisocyanat), 1,3- oder 1,4-Bis(isocyanatomethyl)cyclohexan, 2,4-, oder 2,6-Diisocyanato-1-methylcyclohexan.

Bevorzugt sind 1,6-Diisocyanatohexan, 1-Isocyanato-3,3,5- trimethyl-5-(isocyanatomethyl)cyclohexan, 4,4'-Di(isocyanatocyclohexyl)methan und Toluylendiisocyanat-Isomerengemische. Besonders bevorzugt sind 1,6-Diisocyanatohexan, 1-Isocyanato-3,3,5- trimethyl-5-(isocyanatomethyl)cyclohexan und 4,4'-Di(isocyanatocyclohexyl)methanund 2,4-12,6-Toluylendiisocyanat-Isomerengemische.

Für das erfindungsgemäße Verfahren können solche Diamine zur Reaktion zu den korrespondierenden Diisocyanaten eingesetzt werden, die bevorzugt ohne signifikante Zersetzung in die Gasphase überführt werden können. Besonders geeignet sind hier Diamine, auf Basis von aliphatischen oder cycloaliphatischen Kohlenwasserstoffen mit 2 bis 18 Kohlenstoffatomen. Beispiele hierfür sind 1,6-Diaminohexan, 1-Amino-3,3,5-trimethyl-5-aminomethylcyclohexan (IPDA) und 4,4'-Diaminodicyclohexylmethan. Bevorzugt verwendet wird 1,6-Diaminohexan (HDA).

Ebenfalls können für das erfindungsgemäße Verfahren aromatische Diamine verwendet werden, die bevorzugt ohne Zersetzung in die Gasphase überführt werden können. Beispiele für bevorzugte aromatische Diamine sind Toluylendiamin (TDA), als 2,4- oder 2,6-Isomer oder als Gemisch davon, Diaminobenzol, Napthyldiamin (NDA) und 2,4'-oder 4,4'-Methylen(diphenylamin) (MDA) oder Isomerengemische davon. Bevorzugt sind unter diesen die Diamine, besonders bevorzugt ist 2,4- und/oder 2,6-TDA.

Die Edukte oder auch nur eines von ihnen können zusammen mit einem Inertmedium in den Reaktionsraum eindosiert werden.

Dem erfindungsgemäßen Verfahren kann ein zusätzliches Inertmedium beigesetzt werden. Bei dem Inertmedium handelt es sich um ein Medium, das bei der Reaktionstemperatur gasförmig im Reaktionsraum vorliegt und nicht mit den im Reaktionsverlauf auftretenden Verbindungen reagiert. Das Inertmedium wird im allgemeinen vor der Umsetzung mit Amin und/oder Phosgen vermischt, kann aber auch getrennt von den Eduktströmen zudosiert werden. Beispielsweise können Stickstoff, Edelgase, wie Helium oder Argon, oder Aromaten, wie Chlorbenzol, Dichlorbenzol, Xylol, Kohlenstoffdioxid oder Kohlenstoffmonoxid, verwendet werden. Bevorzugt wird Stickstoff und/oder Chlorbenzol als Inertmedium verwendet.

Im allgemeinen wird das Inertmedium in einer Menge eingesetzt, so dass das Gasvolumen von Inertmedium zu Amin bzw. zu Phosgen mehr als 0,0001 bis 30, bevorzugt mehr als 0,01 bis 15, besonders bevorzugt mehr als 0,1 bis 5 beträgt.

Bevorzugt wird das Inertmedium zusammen mit dem Diamin in den Reaktionsraum eingeführt.

Die Zuführung von Phosgen über den phosgenhaltigen Strom zum Reaktionsraum kann auch so erfolgen, dass man statt eines einzelnen phosgenhaltigen Stroms mehrre phosgenhaltige Teilströme zuführt. In einem solchen Fall werden die phosgenhaltigen Teilströme zu einem gesamten phosgenhaltigen Gesamtstrom addiert, und der Massenbruch des Chlor im phosgenhaltigen Gesamtstrom ergibt sich aus den Massenbrüchen des Chlor in den einzelnen phosgenhaltigen Teilströmen unter Annahme der Molekülerhaltung ohne Reaktion. In diesem Fall wird der so berechnete Wert des Chlor-Massenbruchs im gedanklichen Phosgen-Gesamtstrom verwendet.

Analoges gilt für den Gehalt an Brom.

Derartige Teilströme können in folgender Weise dosiert werden:
- Man kann verschiedene phosgenhaltige Teilströme, beispielsweise rückgeführtes Phosgen und Frisch-Phosgen, vor der Einspeisung zu einem phosgenhaltigen Gesamtstrom vereinigen und in den Reaktionsraum einspeisen.
- Man kann mehrere Teilströme, wobei es sich jeweils um rückgeführtes Phosgen, Frisch-Phosgen oder Gemische daraus handeln kann, an der gleichen Stelle in den Reaktionsraum einspeisen, beispielsweise über mehrere Düsen, die parallel um eine Zentraldüse angeordnet sind, wie es beispielsweise in EP 1449826 A1 beschrieben ist, durch Dosierung über einen Ringspalt, wie sie beispielsweise beschrieben ist in der internationalen Anmeldung WO 2007/028715 mit dem Aktenzeichen PCT/EP2006/065593 und dem Anmeldedatum 23.08.2006, oder durch mehrfache Eindüsung in einen Ringraum zur Vermischung, bevor dieser Strom mit einem über eine Zentraldüse dosierten aminhaltigen Strom vermischt wird.
- Man kann mehrere Teilströme, wobei es sich jeweils um rückgeführtes Phosgen, Frisch-Phosgen oder Gemische daraus handeln kann, an verschiedenen Stellen des Reaktionsraums eindosieren, so daß Phosgen im Verlauf der Reaktion nachdosiert wird.

Mit dem Begriff "Frisch-Phosgen" ist damit ein phosgenhaltiger Strom bezeichnet, der nicht aus einem Phosgenierungsverfahren zurückgeführt worden ist, sondern der nach der Synthese des Phosgen, zumeist aus Chlor und Kohlenmonoxid, keine Reaktionsstufe mit einer Phosgenumsetzung durchlaufen hat, in der mehr als 5 % Umsatz des in der Phosgensynthese hergestellten Phosgen erfolgt.

Werden dem Reaktionsraum ein oder mehrere zusätzliche, gasförmige phosgenfreie oder aminfreie Inertströme zugeführt, dann werden diese in der Ausübung des erfindungsgemäßen Verfahrens wie ein Teilstrom des Phosgen-haltigen Gesamtstromes in der Berechnung des Phosgen-haltigen Gesamtstroms berücksichtigt.

Zur Durchführung des erfindungsgemäßen Verfahrens kann es vorteilhaft sein, die Ströme der Reaktanten vor dem Vermischen vorzuwärmen, üblicherweise auf Temperaturen von 100 bis 600 °C, bevorzugt von 200 bis 450 °C.

In der Aminvorlage wird das Amin bevorzugt zusammen mit einem Inertmedium als Trägergas, wie beispielsweise Stickstoff, in die Gasphase überführt und in die Mischeinheit eingespeist. Das Amin kann aber auch direkt ohne Verwendung eines Inertmediums verdampft werden. Ebenfalls wird Phosgen aus der Phosgenvorlage, gegebenenfalls zusammen mit einem Inertmedium, in die Gasphase übergeführt und in die Mischeinheit geleitet.

Im erfindungsgemäßen Verfahren erfolgt die Vermischung der Reaktanden bevorzugt in einer Mischeinrichtung, die sich durch eine hohe Scherung des durch die Mischeinrichtung geführten Reaktionsstromes auszeichnet. Bevorzugt werden als Mischeinrichtung eine statische Mischeinrichtung oder eine Mischdüse verwendet, die dem Reaktor vorangestellt ist. Besonders bevorzugt wird eine Mischdüse verwendet.

Die Art der Mischung spielt erfindungsgemäß keine Rolle und kann auf beliebige Art und Weise erfolgen, beispielsweise wie beschrieben in EP-B1 699657, EP-A2 1319655, Sp. 1, Z. 54 bis Sp. 2, Z. 24 und Sp. 4, Z. 16 - 40, EP-A1 1275640, Sp. 3, Z. 27 - Sp. 4, Z. 5, EP-A2 1362847, Sp. 2, Z. 19 - Sp. 3, Z. 51 und Sp. 4, Z. 40 - Sp. 5, Z. 12, oder der internationalen Anmeldung WO 2007/028715 mit dem Aktenzeichen PCT/EP2006/065593 und dem Anmeldedatum 23.08.2006, S.2, Z. 23 bis S.11, Z. 22, auf die jeweils im Umfang dieser Offenbarung ausdrücklich Bezug genommen sei.

Erfindungsgemäß wird Phosgen im Überschuss bezüglich Aminogruppen eingesetzt. Üblicherweise liegt ein molares Verhältnis von Phosgen zu Aminogruppen von 1,1 :1 bis 20 : 1, bevorzugt von 1,2 :1 bis 5 :1 vor.

Nach dem Vermischen in der Mischeinheit wird das gasförmige Gemisch aus Phosgen, Amin und gegebenenfalls Inertmedium in den Reaktor geführt, wobei der Reaktor den Reaktionsraum enthält.

Die Umsetzung von Phosgen mit Amin erfolgt in einem Reaktionsraum, der im allgemeinen in einem Reaktor angeordnet ist, d.h. unter Reaktionsraum wird der Raum verstanden, in dem der größte Teil der Umsetzung der Edukte und Zwischenprodukte erfolgt, beispielsweise mindestens 0,5 mol% des eingesetzten Amins zum korrespondierenden Isocyanat, bevorzugt mindestens 1 mol%, besonders bevorzugt mindestens 3 mol%, ganz besonders bevorzugt mindestens 5, insbesondere mindestens 7 und speziell mindestens 10 mol%.

Unter Reaktor wird die technische Vorrichtung verstanden, die den Reaktionsraum enthält. Hierbei kann es sich um alle üblichen, aus dem Stand der Technik bekannten Reaktionsräume handeln, die zur nicht katalytischen, einphasigen Gasreaktion, bevorzugt zur kontinuierlichen nicht-katalytischen, einphasigen Gasreaktion geeignet sind und die den geforderten moderaten Drücken standhalten. Geeignete Materialien für den Kontakt mit dem Reaktionsgemisch sind z.B. Metalle, wie Stahl, insbesondere legierter Stahl, Tantal, Nickel, Nickellegierungen, Silber oder Kupfer, Glas, Keramik, E-maille oder homogene oder heterogene Gemische und Bauteile daraus. Bevorzugt werden Stahlapparate, besonders bevorzugt -reaktoren verwendet. Die Wände des Reaktors können glatt oder profiliert sein. Als Profile eignen sich beispielsweise Ritzen oder Wellen.

Es können im allgemeinen die aus dem Stand der Technik bekannten Reaktorbautypen verwendet werden. Beispiele für Reaktoren sind bekannt aus EP-B1 289840, Sp. 3, Z. 49 - Sp. 4, Z. 25, EP-B1 593334, WO 2004/026813, S. 3, Z. 24 - S. 6, Z. 10, WO 03/045900, S. 3, Z. 34 - S. 6, Z. 15, EP-A1 1275639, Sp. 4, Z. 17 - Sp. 5, Z. 17 und EP-B1 570799, Sp. 2, Z. 1 - Sp. 3, Z. 42, auf die jeweils im Umfang dieser Offenbarung ausdrücklich Bezug genommen sei.

Bevorzugt verwendet werden Rohrreaktoren.

Ebenfalls ist es möglich im wesentlichen quaderförmige Reaktionsräume, bevorzugt Plattenreaktoren bzw. Plattenreaktionsräume zu verwenden. Ein besonders bevorzugter Plattenreaktor weist ein Verhältnis von Breite zu Höhe von mindestens 2 : 1, bevorzugt mindestens 3 : 1, besonders bevorzugt mindestens 5 : 1 und insbesondere mindestens 10 : 1 auf. Die obere Grenze des Verhältnisses von Breite zu Höhe hängt von der gewünschten Kapazität des Reaktionsraums ab und ist prinzipiell nicht begrenzt. Technisch sinnvoll haben sich Reaktionsräume mit einem Verhältnis von Breite zu Höhe bis maximal 5000 : 1, bevorzugt 1000 : 1 erwiesen.

Die Umsetzung von Phosgen mit Amin im Reaktionsraum erfolgt bei Absolutdrücken von mehr als 0,1 bar bis weniger als 20 bar, bevorzugt zwischen 0,5 bar und 15 bar und besonders bevorzugt zwischen 0,7 und 10 bar. Im Fall der Umsetzung von (cyclo)aliphatischen Aminen beträgt der Absolutdruck ganz besonders bevorzugt zwischen 0,7 bar und 5 bar, insbesondere von 0,8 bis 3 bar und speziell 1 bis 2 bar.

Im allgemeinen ist der Druck in den Zuleitungen zur Mischvorrichtung höher, als der vorstehend angegebene Druck im Reaktor. Je nach Wahl der Mischvorrichtung fällt an dieser Druck ab. Bevorzugt ist der Druck in den Zuleitungen um 20 bis 2000 mbar, besonders bevorzugt von 30 bis 1000 mbar höher als im Reaktionsraum.

In einer bevorzugten Ausführungsform besteht der Reaktor aus einem Bündel an Reaktoren. In einer möglichen Ausführungsform muss es sich bei der Mischeinheit nicht um eine eigenständige Vorrichtung handeln, vielmehr kann es vorteilhaft sein, die Mischeinheit in den Reaktor zu integrieren. Ein Beispiel einer integrierten Einheit aus Mischeinheit und Reaktor stellt ein Rohrreaktor mit angeflanschten Düsen dar.

Im allgemeinen ist der Druck in der Aufarbeitungsvorrichtung niedriger als im Reaktionsraum. Bevorzugt ist der Druck um 50 bis 500 mbar, besonders bevorzugt 80 bis 150 mbar, niedriger als im Reaktionsraum.

Gemäß dem erfindungsgemäßen Verfahren erfolgt die Umsetzung von Phosgen mit Amin in der Gasphase. Unter Umsetzung in der Gasphase ist zu verstehen, dass die Umwandlung der Eduktströme und Zwischenprodukte zu den Produkten im gasförmigen Zustand miteinander reagieren und im Verlauf der Reaktion während des Durchgangs durch den Reaktionsraum zu mindestens 95%, bevorzugt zu mindestens 98%, besonders bevorzugt zu mindestens 99%, ganz besonders bevorzugt zu mindestens 99,5%, insbesondere zu mindestens 99,8 und speziell zu mindestens 99,9% in der Gasphase bleiben.

Zwischenprodukte sind dabei beispielsweise die aus den Diaminen gebildeten Monoamino monocarbamoylchloride, Dicarbamoylchloride, Monoamino monoisocyanate und Monoisocyanato monocarbamoylchloride sowie die Hydrochloride der Aminoverbindungen.

Bei dem erfindungsgemäßen Verfahren wird die Temperatur im Reaktionsraum so gewählt, dass sie oberhalb der Siedetemperatur des eingesetzten Diamins, bezogen auf die im Reaktionsraum herrschenden Partialdruckverhältnisse, liegt. Je nach eingesetztem Amin und eingestelltem Druck ergibt sich üblicherweise eine vorteilhafte Temperatur im Reaktionsraum von mehr als 200 °C, bevorzugt mehr als 260 °C und besonders bevorzugt mehr als 300 °C. In der Regel beträgt die Temperatur bis zu 600, bevorzugt bis zu 550 °C.

Die mittlere Kontaktzeit des Umsetzungsgemisches im erfindungsgemäßen Verfahren beträgt im allgemeinen zwischen 0,001 Sekunden und weniger als 5 Sekunden, bevorzugt von mehr als 0,01 Sekunden bis weniger als 3 Sekunden, besonders bevorzugt von mehr als 0,015 Sekunden bis weniger als 2 Sekunden. Im der Umsetzung Fall von (cyclo)aliphatischen Aminen kann die mittlere Kontaktzeit ganz besonders bevorzugt von 0,015 bis 1,5 Sekunden, insbesondere von 0,015 bis 0,5 Sekunden, speziell von 0,020 bis 0,1 Sekunden und oft von 0,025 bis 0,05 Sekunden betragen.

In einer bevorzugten Ausführungsform ist die Strömung im erfindungsgemäßen Verfahren durch eine Bodenstein-Zahl von mehr als 10, bevorzugt mehr als 100 und besonders bevorzugt von mehr als 500 charakterisiert.

In einer bevorzugten Ausführungsform werden die Abmessungen des Reaktionsraums und die Strömungsgeschwindigkeiten so gewählt, dass eine turbulente Strömung, d.h. eine Strömung mit einer Reynolds-Zahl von mindestens 2300, bevorzugt mindestens 2700, für das Reaktionsgemisch vorliegt, wobei die Reynolds-Zahl mit dem hydraulischen Durchmesser des Reaktionsraumes gebildet wird.

Bevorzugt durchläuft das gasförmige Reaktionsgemisch den Reaktionsraum mit einer Strömungsgeschwindigkeit von 10 bis 300 Meter/Sekunde, bevorzugt von 25 bis 250 Meter/Sekunde, besonders bevorzugt 40 bis 230, ganz besonders bevorzugt 50 bis 200, insbesondere mehr als 150 bis 190 und speziell 160 bis 180 Meter/Sekunde. Durch die turbulente Strömung werden eine enge Verweilzeit mit geringer Standardabweichung von meist nicht mehr als 6% wie in EP 570799 beschrieben und eine gute Vermischung erreicht. Maßnahmen, wie beispielsweise die in EP-A-593 334 beschriebene Verengung, die zudem verstopfungsanfällig ist, sind nicht notwendig.

Das Reaktionsvolumen kann über seine Außenfläche temperiert werden. Um Produktionsanlagen mit hoher Anlagenkapazität zu bauen, können mehrere Reaktorrohre parallel geschalten werden. Die Umsetzung kann aber auch bevorzugt adiabat erfolgen. Das bedeutet, dass über die Außenfläche des Reaktionsvolumens nicht mit technischen Maßnahmen Heiz- oder Kühlenergieströme fließen. Bevorzugt findet die Umsetzung adiabat statt.

Das erfindungsgemäße Verfahren wird bevorzugt einstufig durchgeführt. Darunter ist zu verstehen, dass die Vermischung und Umsetzung der Edukte in einem Schritt und in einem Temperaturbereich, bevorzugt in dem vorstehend genannten Temperaturbereich, erfolgt. Ferner wird das erfindungsgemäße Verfahren bevorzugt kontinuierlich durchgeführt.

Nach der Reaktion wird das gasförmige Umsetzungsgemisch bevorzugt bei Temperaturen größer 130 °C mit einem Lösungsmittel gewaschen (Quench). Als Lösungsmittel sind bevorzugt Kohlenwasserstoffe, die gegebenenfalls mit Halogenatomen substituiert sind, geeignet, wie beispielsweise Chlorbenzol, Dichlorbenzol, und Toluol. Als Lösungsmittel wird besonders bevorzugt Monochlorbenzol eingesetzt. Als Lösungsmittel kann auch das Isocyanat eingesetzt werden. Bei der Wäsche wird das Isocyanat selektiv in die Waschlösung übergeführt. Anschließend werden das verbleibende Gas und die erhaltene Waschlösung bevorzugt mittels Rektifikation in Isocyanat, Lösungsmittel, Phosgen und Chlorwasserstoff aufgetrennt. Bevorzugt wird das Isocyanat verwendet.

Nachdem das Reaktionsgemisch im Reaktionsraum umgesetzt wurde, führt man es in die Aufarbeitungsvorrichtung mit Quench. Bevorzugt handelt es sich hier um einen sogenannten Waschturm, wobei aus dem gasförmigen Gemisch das gebildete Isocyanat durch Kondensation in einem inerten Lösungsmittel abgetrennt wird, während überschüssiges Phosgen, Chlorwasserstoff und gegebenenfalls das Inertmedium die Aufarbeitungsvorrichtung gasförmig durchlaufen. Als inertes Lösungsmittel sind bevorzugt Kohlenwasserstoffe, die gegebenenfalls mit Halogenatomen substituiert sind, geeignet, wie beispielsweise Chlorbenzol, Dichlorbenzol, und Toluol. Bevorzugt wird dabei die Temperatur des inerten Lösungsmittels oberhalb der Lösungstemperatur des zum Amin gehörigen Carbamylchlorids im gewählten Quenchmedium gehalten. Besonders bevorzugt wird dabei die Temperatur des inerten Lösungsmittels oberhalb der Schmelztemperatur des zum Amin gehörigen Carbamylchlorids gehalten

Die Wäsche kann beispielsweise in einem Rührbehälter oder in anderen herkömmlichen Apparaturen, z.B. in einer Kolonne oder Mixer-Settler-Apparatur, durchgeführt werden.

Verfahrenstechnisch können für eine Wäsche im erfindungsgemäßen Verfahren alle an sich bekannten Extraktions- und Waschverfahren und -apparate eingesetzt werden, z.B. solche, die in Ullmann's Encyclopedia of Industrial Chemistry, 6th ed, 1999 Electronic Release, Kapitel: Liquid - Liquid Extraction -Apparatus, beschrieben sind. Beispielsweise können dies ein- oder mehrstufige, bevorzugt einstufige Extraktionen, sowie solche in Gleich- oder Gegenstromfahrweise, bevorzugt Gegenstromfahrweise sein.

Ein geeigneter Quench ist beispielsweise bekannt aus EP-A1 1403248, Sp. 2, Z. 39 - Sp. 3, Z. 18, der Europäischen Patentanmeldung mit dem Aktenzeichen 06123629.5 und dem Anmeldedatum 07.11.2006 oder der Europäischen Patentanmeldung mit dem Aktenzeichen 06123621.2 und dem Anmeldedatum 07.11.2006, auf die im Umfang dieser Offenbarung ausdrücklich Bezug genommen sei.

In dieser Quenchzone wird das Reaktionsgemisch, das im wesentlichen aus den Isocyanaten, Phosgen und Chlorwasserstoff sowie Chlor und/oder Brom besteht, intensiv mit der eingedüsten Flüssigkeit vermischt. Die Vermischung erfolgt derart, dass die Temperatur des Reaktionsgemisches ausgehend von 200 bis 500°C um 50 bis 300°C, vorzugsweise 100 bis 250°C abgesenkt wird und das im Reaktionsgemisch enthaltene Isocyanat durch Kondensation vollständig oder teilweise in die eingedüsten Flüssigkeitströpfchen übergeht, während das Phosgen und der Chlorwasserstoff sowie Chlor und/oder Brom im wesentlichen vollständig in der Gasphase verbleiben.

Der Anteil des im gasförmigen Reaktionsgemisch enthaltenen Isocyanats, das in der Quenchzone in die Flüssigphase übergeht, beträgt dabei vorzugsweise 20 bis 100 Gew.-%, besonders bevorzugt 50 bis 99,5 Gew.-% und insbesondere 70 bis 99 Gew.-%, bezogen auf das im Reaktionsgemisch enthaltene Isocyanat.

Das Reaktionsgemisch durchströmt die Quenchzone vorzugsweise von oben nach unten. Unterhalb der Quenchzone ist ein Sammelbehälter angeordnet, in dem die Flüssigphase abgeschieden, gesammelt und, über einen Auslass aus dem Reäktionsraum entfernt und anschließend aufgearbeitet wird. Die verbleibende Gasphase wird über einen zweiten Auslass aus dem Reaktionsraum entfernt und ebenfalls aufgearbeitet.

Der Quench kann beispielsweise erfolgen, wie in der EP 1403248 A1 beschrieben, oder wie in der WO 2005/123665 beschrieben.

Die Flüssigkeitströpfchen werden dazu mittels Ein- oder Zweistoffzerstäüberdüsen, vorzugsweise Einstoffzerstäuberdüsen, erzeugt und erzeugen je nach Ausführungsform einen Sprühkegelwinkel von 10 bis 140°, bevorzugt von 10 bis 120°, besonders bevorzugt von 10° bis 100°.

Die Flüssigkeit, die über die Zerstäuberdüsen eingedüst wird, muss eine gute Löslichkeit für Isocyanate aufweisen. Vorzugsweise werden organische Lösungsmittel eingesetzt. Insbesondere eingesetzt werden aromatische Lösungsmittel, die mit Halogenatomen substituiert sein können. Beispiele für derartige Flüssigkeiten sind Toluol, Benzol, Nitrobenzol, Anisol, Chlorbenzol, Dichlorbenzol (otho, para), Trichlorbenzol, Xylol, Hexan, Diethylisophthalat (DEIP), Tetrahydrofuran (THF), Dimethylformamid (DMF) und deren Gemische, bevorzugt Monochlorbenzol.

In einer besonderen Ausführungsform des erfindungsgemäßen Verfahrens handelt es sich bei der eingedüsten Flüssigkeit um ein Gemisch aus Isocyanaten, ein Gemisch aus Isocyanaten und Lösungsmittel oder um Isocyanat, wobei die jeweils verwendete Quenchflüssigkeit Anteile an Leichtsieder, wie HCI und Phosgen, aufweisen kann. Vorzugsweise wird dabei das Isocyanat eingesetzt, das bei dem jeweiligen Verfahren hergestellt wird. Da durch die Temperatursenkung in der Quenchzone die Reaktion zum Stillstand kommt, können Nebenreaktionen mit den eingedüsten Isocyanaten ausgeschlossen werden. Der Vorteil dieser Ausführungsform liegt insbesondere darin, dass auf eine Abtrennung des Lösungsmittels verzichtet werden kann.

In einer alternativen bevorzugten Ausführungsform handelt es sich bei dem Inertmedium, das zusammen mit mindestens einem der Edukte eingesetzt wird, und bei dem Lösungsmittel, das im Quench eingesetzt wird, um die gleiche Verbindung, ganz besonders bevorzugt wird in diesem Fall Monochlorbenzol verwendet.

Geringe Mengen von Nebenprodukten, die im Isocyanat verbleiben, können mittels zusätzlicher Rektifikation, durch Strippen mit einem Inertgas oder auch Kristallisation, bevorzugt durch Rektifikation vom erwünschten Isocyanat getrennt werden.

In der anschließenden optionalen Reinigungsstufe wird das Isocyanat, bevorzugt durch Rektifikation, vom Lösungsmittel abgetrennt. Ebenfalls kann hier noch die Abtrennung von restlichen Verunreinigungen, umfassend insbesondere Chlor und/oder Brom, aber auch Chlorwasserstoff, Inertmedium und/oder Phosgen, erfolgen, wie beispielsweise beschrieben in DE-A1 10260092.

Aus dem Quench und/oder der Reinigungsstufe werden Stoffströme, die im wesentlichen aus Phosgen und/oder Chlorwasserstoffgas bestehen, aber auch Anteile an Chlor enthalten können, erhalten. Aus zumindest einem Teil dieser Phosgen und/oder Chlorwasserstoffgas sowie Chlor und/oder Brom enthaltenden Stoffströme, die wieder in die Reaktion rückgeführt werden, wird erfindungsgemäß Chlor und/oder Brom vom Phosgen abgetrennt, so daß der Massenbruch von Brom im phosgenhaltigen Strom nach gegebenenfalls durchgeführter Vermischung mit Frisch-Phosgen vor der Vermischung mit dem aminhaltigen Strom erfindungsgemäß weniger als 50 Gew.ppm beträgt.

Es kann eine denkbare Ausführungsform darstellen, wenn der phosgenhaltige Gasstrom einen Massengehalt an Chlor von mindestens 0,05 Gew.ppm aufweist, oder sogar von mindestens 0,1 Gew.ppm, wenn nicht sogar von mindestens 1 Gew.ppm. Denkbar sind auch mindestens 5 Gew.ppm.

Es kann eine denkbare Ausführungsform darstellen, wenn der phosgenhaltige Gasstrom einen Massengehalt an Brom von mindestens 0,005 Gew.ppm aufweist, oder sogar von mindestens 0,01 Gew.ppm, wenn nicht sogar von mindestens 0,1 Gew.ppm. Denkbar sind auch mindestens 0,5 Gew.ppm.

Die Trennung des Chlorwasserstoff und/oder Phosgen und/oder Lösungsmittel und/oder Chlor und/oder Brom enthaltenden Gemisches erfolgt bevorzugt über partielle Kondensation und/oder Rektifikation und/oder Wäsche. Bevorzugt wird die Trennung in einer Kombination von einer Rektifikation und einer Wäsche in beliebiger Reihenfolge durchgeführt.

### Wäsche

Bevorzugte Waschmedien sind die oben als Quenchmedien aufgeführten Lösungsmittel. Besonders bevorzugt werden als Wasch- und als Quenchmedium die gleichen Lösungsmittel eingesetzt.

Bei einer kombinierten Wäsche und Rektifikation wird Phosgen aus dem chlorwasserstoffhaltigen Strom durch Wäsche mit einem Waschmedium, bevorzugt Toluol, Chlorbenzol oder Dichlorbenzol, besonders bevorzugt Chlorbenzol ausgewaschen. Dabei fällt ein mit Phosgen und Chlorwasserstoff sowie in der Regel Chlor beladenes Waschmedium an. Die Abtrennung von Phosgen, Chlorwasserstoff (HCI) und molekularem Chlor (Cl₂) aus diesem beladenen Waschmedium nach der Wäsche erfolgt bevorzugt destillativ.

Die Wäsche wird bei Drücken von 1 bis 10 bar absolut betrieben, bevorzugt von 1 bis 5 bar absolut.

Die Wäsche wird bevorzugt bei Temperaturen von -5 bis -40 °C, bevorzugt -15 bis - 35, besonders bevorzugt -20 bis -30°C betrieben.

Verfahrenstechnisch können für eine solche Wäsche im erfindungsgemäßen Verfahren alle an sich bekannten Absorptionsverfahren und -apparate eingesetzt werden, z.B. solche, die in Ullmann's Encyclopedia of Industrial Chemistry, 6th ed, 2000 Electronic Release, Kapitel: "Absorption" und dort bevorzugt in den Unterkapiteln "Design of Absorption Systems", "Design of Absorption Equipment" und "Design of Desorption Equipment", beschrieben sind. Beispielsweise können dies ein- oder mehrstufige, bevorzugt mehrstufig Absorptionen, sowie solche in Gleich- oder Gegenstromfahrweise, bevorzugt Gegenstromfahrweise sein.

Vorzugsweise werden Ventilboden-, Siebboden-, gepackte oder Füllkörperkolonnen sowie gepulste Kolonnen oder solche mit rotierenden Einbauten eingesetzt. Bevorzugt ist es möglich, die Waschflüssigkeit durch eine Düse fein zu verteilen und mit der Gasphase in Kontakt zu bringen.

Dabei erfolgt erfindungsgemäß die Abtrennung so, dass ein Phosgenstrom anfällt, der, gegebenenfalls nach Vermischung mit Frischphosgen einen Gehalt an Brom von kleiner 50 Gew.ppm enthält.

Dazu wird das Mengenverhältnis von aufzutrennendem Gemisch zu Waschflüssigkeit eingestellt auf 5:1 bis 0,5:1, bevorzugt 3:1 bis 1:1 und besonders bevorzugt 2,5:1 bis 1,5:1.

Als weitere Ausführungsform sei der Einsatz von Ionischen Flüssigkeiten als Waschflüssigkeit erwähnt, wie es beispielsweise beschrieben ist in WO 2006/029788, dort besonders S.2, Z. 39 bis Seite 11, Z. 25.

### Partielle Kondensation

Alternativ oder zusätzlich kann das Chlor und/oder Brom durch eine partielle Kondensation aus dem Phosgen und Chlorwasserstoff-haltigen Strom abgetrennt werden, nach dem Verfahren, wie es bereits in WO 2004/56758 beschrieben ist, dort bevorzugt von S. 11, Z. 14 bis S. 13, Z. 16 sowie im Beispiel. Auf die Offenbarung sei hiermit ausdrücklich Bezug genommen.

Bevorzugt geschieht dies durch Auftrennung eines Stoffgemisches enthaltend Chlorwasserstoff und Phosgen sowie Chlor und/oder Brom, gegebenenfalls Lösungsmittel, Leichtsieder und Inerte, in zunächst mindestens einer partiellen oder vollständigen, bevorzugt partiellen Kondensation des Phosgen und Chlorwasserstoff, sowie gegebenenfalls Lösungsmittel enthaltenden Stroms, dann eine Rektifikation oder Strippung in einer Kolonne zur Entfernung von Chlorwasserstoff und Chlor und/oder Brom aus dem Sumpfprodukt Phosgen und anschließend bevorzugt einer Wäsche des Kopfproduktes Chlorwasserstoff mit dem Prozeßlösungsmittel zur Absorption des Phosgens im Prozeßlösungsmittel durchgeführt wird. Zur Entfernung von Lösungsmittelresten und/oder Chlor und/oder Brom und/oder Chlorwasserstoff aus dem Phosgen kann anschließend deren Nachreinigung mittels Adsorption, beispielsweise auf Aktivkohle, oder durch andere geeignete Methoden erfolgen.

Die partielle Kondensation kann gegebenenfalls mehrstufig und bei verschiedenen Temperatur- und Druckniveaus erfolgen, anschließend kann eine weitere Rektifikation oder Strippung in einer Kolonne zur Entfernung von Chlorwasserstoff und/oder Chlor und/oder Brom aus dem kondensierten Phosgen erfolgen.

Die partielle Kondensation von Phosgen aus dem anfallenden Gemisch, enthaltend Chlorwasserstoff, Phosgen, Chlor und/oder Brom, sowie gegebenenfalls Lösungsmittel und Inerte, erfolgt ein- oder vorzugsweise mehrstufig bei Temperaturen von -40°C, erreichbar mittels Kältemittel, bis 40°C, erreichbar mittels Kühlwasser, je nach dem Druck im Reaktionsteil.

### Rektifikation

Die Rektifikation zur Entfernung von Chlor und/oder Brom und gegebenenfalls Chlorwasserstoff aus dem so erhaltenen, kondensierten Phosgen wird bei einer Sumpftemperatur von 5 bis 150°C, bevorzugt 5 bis 50°C, einem Kopfdruck von 1 bis 35 bar, bevorzugt 1,5 bis 4,0 bar und einer Kopftemperatur von -20 °C bis 30°C, bevorzugt von -10 °C bis 0 °C, durchgeführt.

Die Rektifikation kann in handelsüblichen Destillationskolonnen mit beispielsweise 3 bis 30, bevorzugt 5 bis 25 theoretischen Trennstufen durchgeführt werden.

Alternativ kann das Chlor und/oder Brom und gegebenenfalls der Chlorwasserstoff auch durch Strippen mit einem Inertgas wie Stickstoff, dem Prozeßlösungsmitteldampf, Phosgen oder einem anderen gasförmigen oder zu verdampfenden Stoff aus dem Rückführphosgen entfernt werden.

### Adsorption an Aktivkohle

Der aus einer Herstellungsanlage von Phosgen stammende oder nach der Destillation des Phosgen und Chlorwasserstoff enthaltenden Gemisches erhaltene Phosgenstrom kann bereits einen so geringen Chlorgehalt aufweisen, daß er das erfindungsgemäße Kriterium erfüllt, es kann jedoch auch noch eine weitere Nachbehandlung erforderlich sein.

Eine derartige Nachbehandlung kann bevorzugt durch Absorption von in dem Phosgen enthaltenen Chlor und/oder Brom an Aktivkohle geschehen, wie es beispielsweise beschrieben ist in der US 3331873.

Aktivkohle kann beträchtliche Mengen Chlor und/oder Brom adsorbieren, beispielsweise bis zu 20 Gew%, bevorzugt bis zu 16 Gew% und kann durch Erhitzen auf Temperaturen oberhalb von 70 °C, bevorzugt 150 - 250 °C regeneriert werden.

Die Aktivkohle, die in dem Verfahren eingesetzt werden kann, kann eine beliebige handelsübliche Aktivkohle sein. Die Wirkungsweise hängt dabei zum Teil von einem großen Verhältnis ihrer Oberfläche zu ihrer Masse ab. Die Aktivkohlen sind in verschiedenen Porositätsgraden der einzelnen Teilchen erhältlich. Für das Verfahren sind sowohl solche Aktivkohlen aus Mineralien als auch aus tierischen oder pflanzlichen Stoffen gewonnenen Aktivkohlen geeignet. Eine Aktivkohle mit einer relativ geringen Porenradien, beispielsweise von etwa 2 bis 3,5, bevorzugt 2 bis 3 und besonders bevorzugt etwa 2,5 Å hat sich als besonders wirkungsvoll erwiesen und ist daher zu bevorzugen.

Die Aktivkohle kann in beliebigen Formkörpern vorliegen, beispielsweise als Stränge, Pulver, Tabletten, Granulat, Preßlinge oder Extrudat.

Bevorzugt wird ein gefüllter Turm oder eine gefüllte Säule, wie sie gewöhnlich zum Trennen von Komponenten aus Gemischen mittels Aktivkohle benutzt werden. Auch können andere Apparate, wie mit starken und schnellen Rührem ausgestattete Gefäße mit Filterschichten, verwendet werden. Vorzugsweise sind Vorrichtungen zum Heizen oder Kühlen der Aktivkohle vorgesehen. Das Verfahren selber kann diskontinuierlich oder bevorzugt kontinuierlich durchgeführt werden.

In einer beispielhaften Ausführungsform des Verfahrens wird ein chlor- und/oder bromhaltiger Phosgenstrom in einen Absorptionsturm geleitet, der mit Aktivkohle beschickt und von einem Wärmetauscher umgeben ist, durch den kontinuierlich Kühlsole zirkuliert. Das Rohphosgen tritt in die Säule, die auf eine Temperatur von unter 8 °C, bevorzugt 0 bis -10 °C abgekühlt ist und bei dieser Temperatur gehalten wird, an ihrem unteren Ende ein und strömt durch die Aktivkohle. Das gereinigte Phosgen verläßt die Säule an ihrem oberen Ende oder in dessen Nähe.

Zur Regenerierung kann die verbrauchte Aktivkohle auf eine Temperatur von mindestens 70 °C, bevorzugt 150 - 250 °C erhitzt werden.

Zum Regenerieren kann ein Strom eines unter den Regenerierungsbedingungen inerten Gases, beispielsweise Stickstoff, Argon, Kohlendioxid oder bevorzugt Kohlenmonoxid, mit einer Durchflußgeschwindigkeit von 120 - 370 kg/m² Querschnitt des Aktivkohlebettes und Stunde solange durch die Aktivkohle hindurchgeleitet werden, bis er praktisch phosgenfrei ist, d.h. weniger als 0,05 Vol% Phosgen enthält. Erfindungsgemäß bevorzugt erfolgt die Auftrennung von Phosgen, Lösungsmittel aus dem Quench, also bevorzugt Monochlorbenzol, Chlorwasserstoff und Chlor in den folgenden bevorzugten Kombinationen von Verfahrensschritten, insbesondere einer Kombination aus partieller Kondensation und Wäsche oder einer Kombination aus partieller Kondensation und Rektifikation.

Das aufzutrennende Gemisch ist in der Regel wie folgt zusammengesetzt:
- Lösungsmittel aus Quench 2 - 60 Gew%, bevorzugt 5 bis 40 Gew%, besonders bevorzugt 10 - 30 Gew%,
- Phosgen 20 - 95 Gew%, bevorzugt 40 - 85 Gew%, besonders bevorzugt 60 - 75 Gew%,
- Chlorwasserstoff 1 - 50 Gew%, bevorzugt 2 - 30 Gew%, besonders bevorzugt 5 - 20 Gew% und ganz besonders bevorzugt 5 -15 Gew%,
- Chlor 10 - 10000 Gew.ppm, bevorzugt 100 - 5000 Gew.ppm, besonders bevorzugt 250 - 3000 Gew.ppm und ganz besonders bevorzugt 500 - 2000 Gew.ppm,
- Brom 0,01 -100 Gew.ppm, bevorzugt 0,05 - 50 Gew.ppm, besonders bevorzugt 0,1
- 10 Gew.ppm und ganz besonders bevorzugt 0,2 - 10 Gew.ppm, sowie
- Diisocyanat, dessen Zwischenprodukte und Folgeprodukte (in Summe) 0 - 10 Gew%, bevorzugt 0 - 5 Gew%, besonders bevorzugt 10 Gew.ppm - 3 Gew% und ganz besonders bevorzugt 100 Gew.ppm bis 1 Gew%,
mit der Maßgabe, daß die Summe 100 Gew% ergibt.

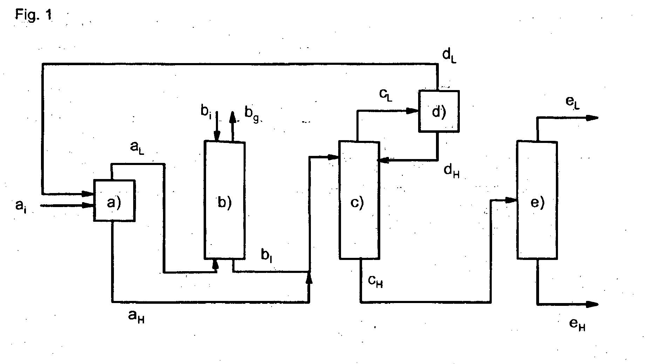

In einer ersten, bevorzugten Ausführungsform, wie sie in Figur 1 dargestellt ist, wird dieses aufzutrennende Gemisch aᵢ in einem Schritt a) in eine partielle Kondensation geführt, aus der ein Kondensat a_{H} erhalten wird, das überwiegend Lösungsmittel, abgereicherte Mengen an Phosgen und Spuren von Chlorwasserstoff und Chlor enthält, sowie ein gasförmiger Strom a_{L}, der überwiegend aus Phosgen, Chlorwasserstoff und Chlor besteht sowie untergeordneten Mengen an Lösungsmittel. Diese partielle Kondensation wird in der Regel betrieben bei einem Druck von 0,1 bis 20 bar abs, bevorzugt 0,2 bis 10, besonders bevorzugt 0,5 bis 5 und ganz besonders bevorzugt 0,8 bis 2 bar abs. Der aus der Kondensation austretende Strom hat beispielsweise eine Temperatur von -5 bis -40 °C, bevorzugt -15 bis -35, besonders bevorzugt -20 bis -30 °C.

Der so erhaltene gasförmige Strom a_{L} wird dann in eine Wäsche b) geführt, in der dieser Strom bevorzugt im Gegenstrom mit einer Waschflüssigkeit bᵢ behandelt wird, wie oben beschrieben. Bevorzugt sind Waschflüssigkeit und Lösungsmittel im aufzutrennenden Gemisch die gleichen. Aus der Wäsche b) wird ein gasförmiger Strom b_{g} erhalten, aus dem Phosgen und Lösungsmittel nahezu vollständig entfernt sind und der im wesentlichen aus Chlorwasserstoff, Chlor und/oder Brom besteht. Ferner kann Waschflüssigkeit beispielsweise durch Mitriß enthalten sein. Flüssige Bestandteile des Stroms b_{g} können durch nachgeschaltete Dephlegmatoren oder Tröpfchenabscheider entfernt werden. Der Strom b_{g} kann dann nach gegebenenfalls weiteren Reinigungsschritten in Verfahren zur Verwertung von Chlorwasserstoff geführt werden, beispielsweise wie beschrieben in DE 10235476 (entspricht US 6916953).

Bei einer solchen Verwertung von Chlorwasserstoff kann es sich bevorzugt um eine Chlorherstellung, besonders bevorzugt um eine Elektrolyse oder ganz besonders bevorzugt um ein Deacon-Verfahren handeln.

Der flüssige Abzug bₗ aus der Waschstufe b) besteht dann überwiegend aus Waschflüssigkeit, in der sich der größte Teil des in die Stufe b) geführten Phosgen aus dem Strom a_{L} befindet. Zusätzlich kann sich auch noch Chlorwasserstoff in geringen Mengen in der Waschflüssigkeit befinden neben Spuren von Chlor.

Die Wäsche ist bevorzugt so ausgestaltet, daß sie 2 bis 25 thermodynamische Stufen, besonders bevorzugt 5 bis 20 und ganz besonders bevorzugt 10 bis 15 thermodynamische Stufen aufweist.

Die Wäsche wird in der Regel betrieben bei einem Druck von 0,1 bis 20 bar abs, bevorzugt 0,2 bis 10, besonders bevorzugt 0,5 bis 5 und ganz besonders bevorzugt 0,8 bis 2 bar abs. Die Temperatur des Waschmediums beträgt beispielsweise von -5 bis -40 °C, bevorzugt -15 bis -35, besonders bevorzugt -20 bis -30 °C.

Das Mengenverhältnis von Reaktionsgemisch zu Waschflüssigkeit beträgt beispielsweise 5:1 bis 0,5:1, bevorzugt 3:1 bis 1:1 und besonders bevorzugt 2,5:1 bis 1,5:1.

Die Ströme bₗ und a_{H} werden dann vereinigt und in eine Rektifikation c) geführt, in der im wesentlichen die Leichtsieder Chlor und Chlorwasserstoff von Phosgen und Waschflüssigkeit abgetrennt werden. In einer bevorzugten Ausführungsform enthält die Rektifikation c) lediglich einen Abtriebsteil, d.h. der Zustrom zur Rektifikation c) wird oberhalb der trennwirksamen Einbauten in die Rektifikation eingespeist. Der Abtriebsteil umfaßt in der Regel 5 bis 30, bevorzugt 10 bis 25 und besonders bevorzugt 15 bis 20 theoretische Trennstufen. In einer weniger bevorzugten Ausführungsform kann die Rektifikation c) zusätzlich auch einen Verstärkungsteil aus 2 bis 10 theoretischen Trennstufen enthalten. Die Rektifikation c) wird in der Regel bei einer Kopftemperatur von 0 bis 25 °C, bevorzugt 5 bis 20 und besonders bevorzugt 10 bis 15 °C, einer Sumpftemperatur von 20 bis 80 °C, bevorzugt 30 bis 70 und besonders bevorzugt 40 bis 60 °C, sowie einem Druck von 1 bis 5 bar abs, bevorzugt 2 bis 3,5 bar abs betrieben.

Die Rektifikation kann beispielsweise bei einem Rücklaufverhältnis 1 bis 20 betrieben werden, bevorzugt 3 bis 15 und besonders bevorzugt 5 bis12.

Der Hochsiederstrom C_{H} besteht im wesentlichen aus Phosgen und Waschflüssigkeit. Der Brüdenstrom c_{L} kann neben Chlorwasserstoff und Chlor noch geringe Mengen an Phosgen und Waschflüssigkeit enthalten.

In einer bevorzugten Ausführungsform wird der Brüdenstrom c_{L} in einer Stufe d) partialkondensiert, das Kondensat d_{H} in die Stufe c) zurückgeführt. Der gasförmige Strom d_{L}, der überwiegend Chlorwasserstoff und Chlor neben wenig Phosgen enthält, wird dann in die Stufe a) geleitet.

Falls das dem Gesamtprozeß zugeführte Frisch-Phosgen einen hohen Chloranteil aufweist, beispielsweise über 2, bevorzugt über 10, besonders bevorzugt über 20, ganz besonders bevorzugt über 50 und insbesondere über 100 Gew.ppm, der im erfindungsgemäßen Verfahren abgetrennt werden soll, so wird dieses chlorhaltige Frisch-Phosgen vor Umsetzung mit dem Amin erfindungsgemäß zumindest teilweise, bevorzugt vollständig über trennwirksame Einbauten einer Rektifikationskolonne geführt. Dies kann beispielsweise erfolgen, indem dieses chlorhaltige Frisch-Phosgen bevorzugt der Stufe c) zugeführt wird, besonders bevorzugt auf der gleichen Trennstufe wie die anderen Ströme, die der Stufe c) zugeführt werden. Dies kann beispielsweise durch Vermischung mit den Strömen bₗ und a_{H} und anschließender Zuführung in Stufe c) erfolgen.

Bevorzugt ist diese Rektifikationskolonne Bestandteil der Auftrennung des Gemischs aus Chlorwasserstoff und Phosgen sowie gegebenenfalls Inertmedium aus der Reaktionsstufe der Gasphasenphosgenierung.

Der Strom c_{H} wird dann einer letzten Rektifikation e) zugeführt, in der Phosgen und die Waschflüssigkeit voneinander getrennt werden. Bevorzugt sollte die Stufe e) zumindest einen Abtriebsteil aus 2 bis 20, bevorzugt 5 bis 15, besonders bevorzugt 6 bis 10-theoretischen Trennstufen aufweisen, so daß der im Sumpf gewonnene Hochsiederstrom e_{H} im wesentlichen aus reiner Waschflüssigkeit besteht und der Leichtsiederstrom e_{L} ohne Rektifikation über trennwirksame Einbauten im Kopf der Rektifikation e) abgezogen wird. In diesem Fall besteht der Strom e_{H} im wesentlichen aus Waschflüssigkeit und Phosgen und kann bevorzugt ohne weitere Aufreinigung in die Phosgenierung rückgeführt werden, da die Waschflüssigkeit in der Gasphasenphosgenierung dann als Inertmedium wirkt.

In einer besonders bevorzugten Ausführungsform weist die Stufe e) sowohl einen Verstärkungsteil aus bevorzugt 0,5 bis 10, bevorzugt 1 bis 5 theoretischen Trennstufen als auch einen Abtriebsteil aus 2 bis 20, bevorzugt 5 bis 15 und besonders bevorzugt 6 - 10 theoretischen Trennstufen auf und wird bei einer Kopftemperatur von 5 bis 80 °C, bevorzugt 10 bis 60 und besonders bevorzugt 20 bis 40 °C, einer Sumpftemperatur von 100 bis 200 °C, bevorzugt 130 bis 180 und besonders bevorzugt 150 bis 175 °C, sowie einem Druck von 1 bis 5 bar abs, bevorzugt 2 bis 3,5 bar abs betrieben.

Dabei wird die Rektifikation der Stufe e) beispielsweise bei einem Rücklaufverhältnis 0,1 bis 10 betrieben, bevorzugt 0,2 bis 5 und besonders bevorzugt 0,5 bis 2.

Der dabei erhaltene Brüdenstrom e_{L} besteht im wesentlichen aus reinem Phosgen, das dann bevorzugt ohne weitere Aufreinigung in die Phosgenierung eingesetzt werden kann. Der Hochsiederstrom e_{H} besteht im wesentlichen aus reiner Waschflüssigkeit, die dann bevorzugt ohne weitere Aufreinigung in die Wäsche b) und/oder den Quench eingesetzt werden kann.

In einer zweiten Ausführungsform werden alle Schritte der ersten Ausführungsform verwirklicht, mit dem Unterschied, daß die Destillationsstufen c) und e) gemeinsam in einer Trennwandkolonne vereinigt sind. Diese Trennwandkolonne ist bevorzugt so ausgeführt, daß die Zulaufseite lediglich einen Abtriebsteil und die Ablaufseite ausschließlich einen Verstärkungsteil aufweist. Die Trennwand trennt dann die Gasräume von Zulauf- und Ablaufseite voneinander.

Der Gasraum der Zulaufseite ist dann wiederum bevorzugt mit einer partiellen Kondensation d) verbunden. Bei einer solchen Ausführungsform wird der Strom c_{L} dem Gasraum der Zulaufseite entnommen, der Strom e_{L}, der im wesentlichen aus Phosgen besteht, dem Gasraum der Ablaufseite und der Strom e_{H}, der im wesentlichen aus der Waschflüssigkeit besteht, dem gemeinsamen Sumpf von Ablauf- und Zulaufseite.

In einer dritten alternativen Ausführungsform werden wiederum alle Merkmale der ersten Ausführungsform verwirklicht und die Destillationsstufen c) und e) ebenfalls gemeinsam in einer Trennwandkolonne vereinigt. Diese Trennwandkolonne ist dabei jedoch bevorzugt so ausgeführt, daß sowohl die Zulaufseite als auch die Ablaufseite beide einen Abtriebsteil und einen Verstärkungsteil aufweisen. Leichtsieder- und Hochsiederraum sind dann jeweils über trennwicksame Einbauten miteinander verbunden. Die Trennwand trennt Zulauf- und Ablaufseite voneinander.

Der gemeinsame Gasraum ist wiederum bevorzugt mit einer partiellen Kondensation d) verbunden. Bei einer solchen Ausführungsform wird der Strom c_{L} dem gemeinsamen Gasraum entnommen, der Strom e_{L}, der im wesentlichen aus Phosgen besteht, wird nunmehr auf der Ablaufseite als Mittelsieder entnommen und der Strom e_{H}, der im wesentlichen aus der Waschflüssigkeit besteht, dem gemeinsamen Sumpf von Ablauf- und Zulaufseite.

Erfindungsgemäß bevorzugt sind solche Kombinationen von Verfahrensschritten zur Auftrennung von Phosgen, Lösungsmittel aus dem Quench bzw. Waschflüssigkeit aus dem Schritt b), Chlorwasserstoff und Chlor enthaltenden Gemischen, in denen die Waschflüssigkeit, bevor sie in die Wäsche b) und/oder in den Quench rückgeführt wird, mindestens einmal rektifikativ über mindestens eine theoretische Destillationsstufe aufgereinigt worden ist.

Falls das dem Gesamtprozeß zugeführte Frisch-Phosgen einen hohen Chloranteil und/oder Bromanteil, das heißt oberhalb der oben angegeben Grenzwerte, aufweist, der im erfindungsgemäßen Verfahren abgetrennt werden soll, so sind erfindungsgemäß weiterhin bevorzugt solche Kombinationen von Verfahrensschritten, in denen das Phosgen, bevor es in die Phosgenierung geführt wird, mindestens einmal rektifikativ über mindestens eine theoretische Destillationsstufe aufgereinigt worden ist.

Der erfindungsgemäß niedrige Gehalt an Brom kann bevorzugt wie folgt erreicht werden:
1) Das dem Verfahren zugeführte Frisch-Phosgen weist den geforderten niedrigen Bromgehalt auf.
   Erreicht werden können die niedrigen Chlor- und Bromgehalte durch eine Behandlung des dem erfindungsgemäßen Verfahren zugeführten Frisch-Phosgens, beispielsweise mit Aktivkohle, oder durch eine spezielle Verfahrensführung in der Synthese des Phosgen aus Chlor und Kohlenmonoxid.
   Häufig wird Phosgen durch Kontaktieren von Kohlenstoffmonoxid mit molekularem Chlor (Cl₂) an geeigneten Trägern, bevorzugt Aktivkohle, hergestellt. Da die Bildung von Phosgen stark exotherm ist, erfolgt diese bevorzugt in Rohrbündelreaktoren, in denen die Trägerschüttung durch die exotherme Reaktion auf Temperaturen bis zu 400 °C erhitzt wird, wobei die Temperatur jedoch beim Durchgang durch die Rohre auf 40 bis 150 °C absinkt. Die freigesetzte Reaktionswärme wird dabei durch geeignete Wärmeträgermedien, beispielsweise Wasser, abgeführt. Dabei liegt zumeist normaler Druck oder leichter Überdruck an.
   Erfolgt die Herstellung von Phosgen mit einem stöchiometrischen Überschuß von Kohlenstoffmonoxid und ausreichend langer Verweilzeit, so wird in der Regel eine vollständige Umsetzung des Chlor erreicht. Das auf diese Wiese erhaltene Phosgen enthält noch geringe Mengen Kohlenstoffmonoxid. Dies hat auf die Anwendung in der Phosgenierung jedoch keine nachteilige Auswirkung, da das enthaltene Kohlenmonoxid als Inertgas in der Gasphasenphosgenierung fungiert.
2) Weist das Frisch-Phosgen einen relativ hohen Chlorgehalt auf, beispielsweise über 2, bevorzugt über 10, besonders bevorzugt über 20, ganz besonders bevorzugt über 50 und insbesondere über 100 Gew.ppm, so kann das Frisch-Phosgen erfindungsgemäß nicht direkt in die Umsetzung mit dem Amin geführt werden, sondern wird zunächst zumindest teilweise, bevorzugt vollständig in die Trennung des aus der Phosgenierung erhaltenen, Phosgen und Chlorwasserstoff enthaltenden Gemisches eingeleitet, wo Reste von Chlor und/oder Brom zusammen mit Chlorwasserstoff vom Phosgen abgetrennt werden. Dies stellt eine bevorzugte Ausführungsform der vorliegenden Erfindung dar.
   Dies ist vorteilhaft, da dadurch das durch Spaltung des Phosgen entstandene oder aus der Herstellung des Phosgen stammende Chlor vor der Phosgenierung abgetrennt werden kann.
   Der Chlorwasserstoff-/Phosgentrennung kann eine Adsorbtionseineinheit, bevorzugt ein Aktivkohlefilter, im aus der Trennung abgeführten Chlorwasserstoffsstrom nachgeschaltet werden, in dem Spuren des Waschmediums aus dem anfallenden chlorwasserstoff entfernt werden.
   Die nachdem Gasphasenphosgenierungsverfahren hergestellten Diisocyanat eignen sich vorzüglich zur Herstellung von Polyisocyanaten zur Verwendung in Urethan-,Iso-cyanurat-, Amid- und/oder Harnstoffgruppen enthaltenden Kunststoffen nach dem Polyisocyanat-Polyadditionsverfahren. Sie finden ferner Verwendung zur Herstellung von mit Urethan-, Biuret- und/oder Isocyanuratgruppen modifizierten Polyisocyanatmischungen. Derartige Polyisocyanatmischungen aus aliphatischen oder cycloaliphatischen Diisocyanaten werden insbesondere zur Herstellung von lichtbeständigen Polyurethanlacken und -überzügen sowie in thermoplastischen Polyurethanen verwendet.

## Patentansprüche

1. Verfahren zur Herstellung von Diisocyanaten durch Umsetzung der korrespondierenden Diamine mit Phosgen im stöchiometrischen Überschuß von Phosgen in mindestens einer Reaktionszone,
wobei man die Reaktionsbedingungen so wählt, daß zumindest die Reaktionskomponenten Diamin, Diisocyanat und Phosgen bei diesen Bedingungen gasförmig sind, und
wobei man der Reaktionszone mindestens einen diaminhaltigen und mindestens einen phosgenhaltigen Gasstrom zuführt,
wobei der Massenbruch von Brom im phosgenhaltigen Strom weniger als 50 Gew.ppm beträgt.

2. Verfahren gemäß Anspruch 1, **dadurch gekennzeichnet, daß** dem diaminhaltigen und/oder phosgenhaltigen Gasstrom mindestens ein Inertmedium zusetzt, so daß das Gasvolumen von Inertmedium zu Amin bzw. zu Phosgen mehr als 0,0001 bis 30 beträgt.

3. Verfahren gemäß einem der vorstehenden Ansprüche, **dadurch gekennzeichnet, daß** die Umsetzung von Phosgen mit Diamin im Reaktionsraum bei Absolutdrücken von mehr als 0,1 bar bis weniger als 20 bar erfolgt.

4. Verfahren gemäß einem der vorstehenden Ansprüche, **dadurch gekennzeichnet, daß** die Umsetzung von Phosgen mit Diamin im Reaktionsraum bei Temperaturen von mehr als 200 °C bis zu 600 °C erfolgt.

5. Verfahren gemäß einem der vorstehenden Ansprüche, **dadurch gekennzeichnet, daß** die mittlere Kontaktzeit des Umsetzungsgemisches zwischen 0,001 Sekunden und weniger als 5 Sekunden beträgt.

6. Verfahren gemäß einem der vorstehenden Ansprüche, **dadurch gekennzeichnet, daß** das molare Verhältnis von Phosgen zu Aminogruppen von 1,1 :1 bis 20 : 1 beträgt.

7. Verfahren gemäß einem der vorstehenden Ansprüche, **dadurch gekennzeichnet, daß** die Strömung im Reaktionsraum eine Bodensteinzahl von mehr als 10 aufweist.

8. Verfahren gemäß einem der vorstehenden Ansprüche, **dadurch gekennzeichnet, daß** der Massenbruch von Chlorwasserstoff im phosgenhaltigen Strom nach einer gegebenenfalls durchgeführter Vermischung mit Frisch-Phosgen vor der Vermischung mit dem aminhaltigen Strom weniger als 15 Gew.-% beträgt.

9. Verfahren gemäß Anspruch 2, **dadurch gekennzeichnet, daß** das Inertmedium Chlorbenzol ist.

10. Verfahren gemäß einem der vorstehenden Ansprüche, **dadurch gekennzeichnet, daß** das Isocyanat ausgewählt ist aus der Gruppe bestehend aus 1,6-Diisocyanatohexan, 1-Isocyanato-3,3,5- trimethyl-5-(isocyanatomethyl)cyclohexan, 4,4'-Di(isocyanatocyclohexyl)methan und 2,4 /2,6-Toluylendiisocyanat-Isomerengemischen.

11. Verfahren gemäß einem der vorstehenden Ansprüche, **dadurch gekennzeichnet, daß** man Frisch-Phosgen mit einem Anteil an molekularem Chlor (Cl₂) über 2 Gew.ppm vor Umsetzung mit dem Amin erfindungsgemäß zumindest teilweise zur Abtrennung des Chlors vom Phosgen über trennwirksame Einbauten einer Rektifikationskolonne führt.

12. Verfahren gemäß Anspruch 11, **dadurch gekennzeichnet, daß** die Rektifikationskolonne Bestandteil der Auftrennung eines Gemischs aus Chlorwasserstoff und Phosgen sowie gegebenenfalls Inertmedium ist.

13. Verwendung von Phosgen mit einem Gehalt an molekularem Brom (Br₂) von weniger als 50 Gew.ppm in der Herstellung von Diisocyanaten aus Diaminen in einer Gasphasenphosgenierung.

## Claims

1. A process for preparing diisocyanates by reacting the corresponding diamines with a stoichiometric excess of phosgene in at least one reaction zone, wherein the reaction conditions are selected so that at least the reaction components diamine, diisocyanate and phosgene are gaseous under these conditions and
at least one diamine-comprising gas stream and at least one phosgene-comprising gas stream are fed into the reaction zone,
with the mass fraction of bromine in the phosgenecomprising stream being less than 50 ppm by weight.

2. The process according to claim 1, wherein at least one inert medium is added to the diamine-comprising and/or phosgene-comprising gas stream so that the gas volume ratio of inert medium to amine or to phosgene is from > 0.0001 to 30.

3. The process according to either of the preceding claims, wherein the reaction of phosgene with diamine in the reaction space is carried out at absolute pressures of from > 0.1 bar to < 20 bar.

4. The process according to any of the preceding claims, wherein the reaction of phosgene with diamine in the reaction space is carried out at temperatures of from > 200°C to 600°C.

5. The process according to any of the preceding claims, wherein the mean contact time of the reaction mixture is from 0.001 second to < 5 seconds.

6. The process according to any of the preceding claims, wherein the molar ratio of phosgene to amino groups is from 1.1:1 to 20:1.

7. The process according to any of the preceding claims, wherein the flow in the reaction space has a Bodenstein number of more than 10.

8. The process according to any of the preceding claims, wherein the mass fraction of hydrogen chloride in the phosgene-comprising stream after any mixing with fresh phosgene and before mixing with the amine-comprising stream is less than 15% by weight.

9. The process according to claim 2, wherein the inert medium is chlorobenzene.

10. The process according to any of the preceding claims, wherein the isocyanate is selected from the group consisting of 1,6-diisocyanatohexane, 1-isocyanato-3,3,5-trimethyl-5-(isocyanatomethyl)-cyclohexane, 4,4'-di(isocyanato-cyclohexyl)methane and tolylene 2,4-/2,6-diisocyanate isomer mixtures.

11. The process according to any of the preceding claims, wherein fresh phosgene having a proportion of molecular chlorine (Cl₂) above 2 ppm by weight is, according to the invention, at least partly passed over separation-active internals of a rectification column to separate off the chlorine from the phosgene before it is reacted with the amine.

12. The process according to claim 11, wherein the rectification column is a constituent of the separation of a mixture comprising hydrogen chloride and phosgene and possibly inert medium.

13. The use of phosgene having a content of molecular bromine (Br₂) of less than 50 ppm by weight in the preparation of diisocyanates from diamines in a gas-phase phosgenation.

## Revendications

1. Procédé pour la production de diisocyanates par mise en réaction de diamines correspondantes avec du phosgène en excès stoechiométrique de phosgène dans au moins une zone de réaction,
dans lequel on choisit les conditions réactionnelles de manière qu'au moins les composants réactionnels diamine, diisocyanate et phosgène soient gazeux dans ces conditions, et
on envoie à la zone de réaction au moins un courant gazeux contenant de la diamine et au moins un courant gazeux contenant du phosgène,
la fraction massique de brome dans le courant contenant du phosgène étant inférieure à 50 ppm en poids.

2. Procédé selon la revendication 1, **caractérisé en ce qu'**on ajoute au moins un milieu inerte au courant gazeux contenant de la diamine et/ou au courant gazeux contenant du phosgène, de sorte que le volume de gaz du milieu inerte par rapport à l'amine ou au phosgène vaut de plus de 0,0001 à 30.

3. Procédé selon l'une quelconque des revendications précédentes, **caractérisé en ce que** la réaction du phosgène avec la diamine s'effectue dans la chambre de réaction sous des pressions absolues de plus de 0,1 bar à moins de 20 bars.

4. Procédé selon l'une quelconque des revendications précédentes, **caractérisé en ce que** la réaction du phosgène avec la diamine s'effectue dans la chambre de réaction à des températures de plus de 200 °C à 600 °C.

5. Procédé selon l'une quelconque des revendications précédentes, **caractérisé en ce que** le temps de contact moyen du mélange réactionnel est compris entre 0,001 seconde et moins de 5 secondes.

6. Procédé selon l'une quelconque des revendications précédentes, **caractérisé en ce que** le rapport molaire du phosgène aux groupes amino vaut de 1,1 : 1 à 20 : 1.

7. Procédé selon l'une quelconque des revendications précédentes, **caractérisé en ce que** l'écoulement dans la chambre de réaction présente un nombre de Bodenstein de plus de 10.

8. Procédé selon l'une quelconque des revendications précédentes, **caractérisé en ce que** la fraction massique de chlorure d'hydrogène dans le coutant contenant du phosgène après un mélange avec du phosgène neuf, éventuellement effectué, avant le mélange avec le courant contenant de l'amine, est inférieure à 15 % en poids.

9. Procédé selon la revendication 2, **caractérisé en ce que** le milieu inerte est le chlorobenzène.

10. Procédé selon l'une quelconque des revendications précédentes, **caractérisé en ce que** l'isocyanate est choisi dans le groupe constitué par le 1,6-diisocyanatohexane, le 1-isocyanato-3,3,5-triméthyl-5-(isocyanatométhyl)cyclohexane, le 4,4'-di(isocyanatocyclohexyl)méthane et des mélanges d'isomères 2,4-/2,6-toluylènediisocyanate.

11. Procédé selon l'une quelconque des revendications précédentes, **caractérisé en ce qu'**on envoie du phosgène neuf, ayant une teneur en chlore moléculaire (Cl₂) de plus de 2 ppm en poids, avant la réaction avec l'amine, selon l'invention au moins en partie pour la séparation du chlore d'avec le phosgène, sur des inserts efficaces pour la séparation, d'une colonne de rectification.

12. Procédé selon la revendication 11, **caractérisé en ce que** la colonne de rectification est un composant du fractionnement d'un mélange de chlorure d'hydrogène ainsi qu'éventuellement de milieu inerte.

13. Utilisation de phosgène ayant une teneur en brome moléculaire (Br₂) de moins de 50 ppm en poids, dans la production de diisocyanates à partir de diamines dans une phosgénation en phase gazeuse.
